(19) Europäisches Patentamt
European Patent Office
Office européen des brevets

(11) Veröffentlichungsnummer: **0 004 061**
**B1**

# (12) EUROPÄISCHE PATENTSCHRIFT

(45) Veröffentlichungstag der Patentschrift: **19.05.82**

(51) Int. Cl.³: **G 01 N 1/30,** C 09 B 23/10, C 07 D 413/06

(21) Anmeldenummer: **79100663.8**

(22) Anmeldetag: **06.03.79**

## (54) Verfahren zur Bestimmung leukämischer Zellen.

(30) Priorität: **09.03.78 CH 2550/78**

(43) Veröffentlichungstag der Anmeldung: **19.09.79 Patentblatt 79/19**

(45) Bekanntmachung des Hinweises auf die Patenterteilung: **19.05.82 Patentblatt 82/20**

(84) Benannte Vertragsstaaten: **BE CH DE FR GB IT NL SE**

(56) Entgegenhaltungen:
RESEARCH DISCLOSURE, Nr. 135, Juli 1975 HAVANT, HAMPSHIRE, U.K.
W. S. GAUGH, et al.: "Methine Dye scintillators" Seiten 31—35, Nr. 13.507

CHEMICAL ABSTRACTS, Vol. 89, Nr. 1, 3. Juli 1978
Columbus, Ohio U.S.A.
J.E. VALINSKY et al.: "Merocyanine 540 as a fluorescent probe of membranes: selective staining of leukemic and immature hemopoietic cells" Seite 251, Spalte 2, Zusammenfassung Nr. 27.10t

(73) Patentinhaber: **Merck Patent Gesellschaft mit beschränkter Haftung**
**Frankfurter Strasse 250**
**D-6100 Darmstadt (DE)**

(72) Erfinder: **Schäfer, Rolf, Dr. rer. nat.**
**Grabenmattstrasse 37**
**CH-4133 Pratteln (CH)**

EP 0 004 061 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Bestimmung leukämischer Zellen

Die Erfindung betrifft ein Verfahren, mit dem durch selektive Anfärbung leukämische Blutzellen von normalen Leukozyten differenziert werden können, sowie die Verwendung von Fluorochromfarbstoffen zur selektiven Färbung leukämischer Zellen.

Die bekannten Färbmethoden von Leukozyten sind unspezifisch, weil normale und leukämische Zellen damit angefärbt werden. Für eine Leukämiediagnose war man bisher darauf angewiesen, durch Bestimmung der Anzahl von Leukozyten des in den Gefässen zirkulierenden Blutes die pathogene Erhöhung derselben nachzuweisen. Die Leukämiediagnose über die Bestimmung veränderter enzymatischer Aktivitäten von Leukämiezellen ist zeitraubend und apparativ aufwendig.

Der Erfindung liegt die Aufgabe zugrunde, leukämische Blutzellen durch spezifische Färbung von normalen Leukozyten, die nicht gefärbt werden, zu differenzieren.

Die Basis der spezifischen Anfärbbarkeit leukämischer Zellen ist durch die pathogene Änderung der Permeabilität von Leukozytenmembranen bedingt. Im Gegensatz hierzu unterliegen normale Leukozyten nicht diesem Verhalten, so daß eine Differenzierung durch eine selektive Färbung möglich ist.

Es wurde gefunden, daß man das unterschiedliche Verhalten von normalen Leukozyten und leukämischen Zellen gegenüber eine bestimmten Cyaninfarbstoffgruppe (Fluorochrome) zu einer zuverlässigen Leukämiediagnose verwenden kann.

Gegenstand der Erfindung ist ein Verfahren zur Bestimmung leukämischer Zellen, das dadurch gekennzeichnet ist, daß man Leukozyten mit einem Farbstoff der allgemeinen Formel

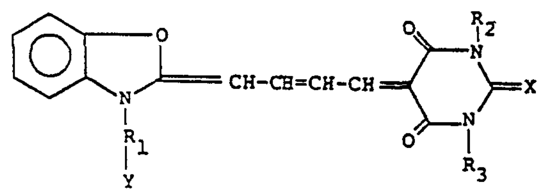

worin

X = S oder O

Y = Sulfo, Sulfoxy, Sulfino, Sulfinoxy, Phosphonoxy, Carboxy sowie die entsprechenden Salze

$R_1$ = Alkylen mit 1—15 C-Atomen

$R_2$ und $R_3$ = Alkylrest mit 1—10 C-Atomen, die auch verzweigt sein können, bedeutet

inkubiert und fluoreszenzmikroskopisch auswertet.

Ferner umfaßt der Gegenstand der Erfindung die Verwendung mindestens eines Farbstoffs der allgemeinen Formel zur selektiven Färbung leukämischer Zellen.

Bevorzugte Farbstoffe zur Durchführung des erfindungsgemäßen Verfahrens sind Farbstoffe der allgemeinen Formel, worin X = O, Y = Sulfonsäurerest, $R_1$ = Alkylen mit 1—9 C-Atomen und $R_2$ und $R_3$ = linearer oder verzweigter Alkylrest mit bis zu 4 C-Atomen bedeutet, vorzugsweise das 3(2H)-Benzoxazolnonansulfonsäure-2-[4-(1,3-dimethyltetrahydro-2,4,6-trioxo-5(2H)-pyrimidinyliden-2-butenyliden]-Natriumsalz, d.H. ein Farbstoff der allgemeinen Formel, worin X = O, Y = Sulfonat, $R_1$ = Nonyl und $R_2$ und $R_3$ = Methyl bedeutet.

Zur Durchführung des Verfahrens werden Leukozyten in isotonischer Salzlösung mit 1,5 bis 30% autologem Plasma suspendiert und 15 Minuten lang mit 10 bis 30 μg/ml des Farbstoffs inkubiert. Nach der üblichen Aufarbeitung, d.h. Zentrifugieren und Waschen, werden die Leukozyten fluoreszenzmikroskopisch ausgewertet. Gegebenenfalls kann im Anschluß an die Färbung mit dem Fluorochrom eine Gegenfärbung mit Trypanblau erfolgen, um die Fluoreszenz aller toten Zellen zu löschen.

Zur Schnellbestimmung leukämischer Leukozyten ist es auch möglich, das erfindungsgemäße Färbeverfahren mit einem Tropfen Blut auf einem Objektträger durchzuführen, wobei die erforderlichen Farbstoffe auf Deckgläschen aufgebracht sein können.

Durch die Verwendung der beschriebenen Fluorochrome ergeben sich folgende Vorteile in der Leukämiediagnose: Die Einfachheit der Methode erlaubt die schnelle routinemäßige Untersuchung ganzer Populationen mittels Cytofluorographie auf Leukämie; die Methode gestattet eine zuverlässige selektive Färbung bei allen bekannten Leukämieformen, selbst bei denjenigen mit ungesicherter Diagnose; leukämische Zellen, die sich morphologisch nicht von Normalleukozyten unterscheiden, sondern nur eine geänderte Membrankonstitution besitzen, werden angefärbt. Dies eröffnet den weit gespannten Bereich von Leukämiefrühdiagnose und Remissions-stadien.

## Beispiel 1

Leukozyten aus Humanblut ($10^7$ Zellen/ml) werden in isotonischer Salzlösung mit 2,5% autologem Plasma suspendiert und in Gegenwart von 25 μg/ml 3(2H)-Benzoxazolnonansulfonsäure-2-[4-(1,3-dimethyltetrahydro-2,4,6-trioxo-5(2H)-pyrimidinyliden-2-butenyliden] während 15 Minuten in einem

offenen Röhrchen bei Raumtemperatur im Dunkeln inkubiert. Danach wird eine Trypanblaugegenfärbung (0,4% Trypanblau) in der gleichen Inkubationslösung gemacht, um die Fluoreszenz aller toten Zellen, die, ob leukämisch oder normal, die freie Permeabilität des Fluorochroms zulassen, für die Auswertung zu löschen. Die Färbereaktion wird durch zweifaches Waschen der Zellen (Zentrifugationen bei 800 × g und 4° Celsius) mit isotonischer Salzlösung in Gegenwart von 2,5% autologem Plasma beendet. Die Zellen werden anschließend im Fluoreszenz-Dunkelfeldmikroskop unter 1000-facher Vergrößerung (Ölimmersionsoptik) mit einer Fluoreszenzanregung von 500 nm (Hg-arc-Lampe) durch einen Leitz K 590 Filter oder ein äquivalentes Filter ausgewertet. Sind dabei fluoreszeierende Zellen zu erkennen, so besteht Verdacht auf Leukämie.

Beispiel 2

Frischblut wird mit 20 Einheiten Heparin pro ml Blut versetzt. Zur heparinisierten Blutprobe wird 1/5 Volumen (bezogen auf das Blutvolumen) Dextranlösung (60 g/l Dextran T 500 (Pharmacia) in isotonischer Salzlösung) zugegeben. Die Probe wird anschließend eine Stunde bei 37°C, inkubiert, wobei die agglutinierten Erythrocyten auf den Boden des Inkubationsröhrchens absinken.

Nach der Inkubation wird der leukozytenreiche Plasmaüberstand vorsichtig abpipettiert und 15 Minuten bei 1000 × g und Raumtemperatur zentrifugiert. Der Überstand wird verworfen und das Pellet (Leukozyten) wird in isotonischer Salzlösung mit 2% autologem Plasma vorsichtig suspendiert. $10^5$—$10^7$ Leukozyten in 0,9 ml Lösung werden mit 0,1 ml eines Farbstoffs der allgemeinen Formel, worin X = S, Y = COOH, $R_1 = CH_2$ und $R_2$ und $R_3 = C_{10}H_{21}$ bedeuten (10 µg/ml Farbstoff in isotonischer Kochsalzlösung) versetzt und während 15 Minuten in einem offenen Röhrchen bei Raumtemperatur und Tageslicht inkubiert. Die Färbung wird durch Zentrifugation der Zellen beendet (100 × g, 15 Minuten bei Raumtemperatur). Die Zellen werden 2 × gewaschen, zentrifugiert und anschließend in isotonischer Kochsalzlösung mit 2% autologem Plasma resuspendiert.

Um die Fluoreszenz toter Zellen (normal oder leukämisch), die durch Präparationsschritte entstehen können, in die der Farbstoff in jedem Falle eindringt, zu löschen, werden die Zellen mit 1 Volumen Trypanblau (0,8% Farbstoff in isotonischer Kochsalzlösung mit 2% autologem Plasma) versetzt und nach ca. 10 Sekunden zentrifugiert und gewaschen. Anschließend werden die Proben analog Beispiel 1 ausgewertet.

Es werden analoge Untersuchungsergebnisse erzielt, wenn man anstelle von 2% autologem Plasma mit 30% autologem Plasma in isotonischer Salzlösung arbeitet.

Beispiel 3

Das Verfahren wird analog Beispiel 1 durchgeführt, als Fluorochrom dient ein Farbstoff der allgemeinen Formel, worin X = O, Y = $PO_4H_2$, $R_1 = C_{15}H_{30}$ und $R_2$ und $R_3 = C_4H_9$ bedeuten, in einer Konzentration von 30 µg/ml. Die isotonische Salzlösung enthält 10% autologes Plasma. Auch damit werden analoge Ergebnisse unter dem Fluoreszensmikroskop erzielt.

Beispiel 4

Ein Tropfen Blut wird auf einen Objektträger gegeben und ein etwa gleichgroßes Deckglas aufgelegt, das mit dem Fluorochrom beschichtet ist. Nach einigen Minuten wird das Präparat unter dem Fluoreszenzmikroskop durch Auszählung der fluoreszierenden, d.h. leukämischen Leukozyten ausgewertet.

Der gleiche Test wird mit einem Deckglas wiederholt, das sowohl mit dem Fluorochrom als auch mit Trypanblau beschichtet ist. Die Auswertung erfolgt auf die gleiche Weise.

Zur Beschichtung der Deckgläser werden die Farbstoffe in Äthanol, das gegebenenfalls etwas Wasser enthält, gelöst und auf das Glas aufgebracht. Nach dem Verdunsten des Lösungsmittels sind die Deckgläser gebrauchsfertig. Sie enthalten 0,1—200 mg Fluorochrom pro $m^2$ bzw. zusätzlich noch 0,1—10 g Trypanblau pro $m^2$.

**Patentansprüche**

1. Verfahren zur Bestimmung leukämischer Zellen, dadurch gekennzeichnet, daß man Leukozyten mit einem Farbstoff der allgemeinen Formel

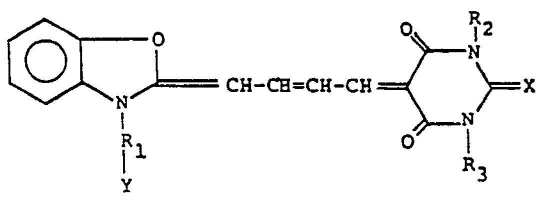

worin

X = O oder S

Y = Sulfo, Sulfoxy, Sulfino, Sulfinoxy, Phosphonoxy, Carboxy sowie die entsprechenden Salze
$R_1$ = Alkylen mit 1—15 C-Atomen
$R_2$ und $R_3$ = Alkylrest mit 1—10 C-Atomen, der auch verzweigt sein kann, bedeutet
inkubiert und fluoreszenzmikroskopisch auswertet.

2. Verfahren nach Anspruch 1, dadurch gekennzeichnet, daß man mit einem Farbstoff der allgemeinen Formel inkubiert, worin X = O, Y = Sulfonsäurerest, $R_1$ = Alkylen mit 1—9 C-Atomen und $R_2$ und $R_3$ linearer oder verzweigter Alkylrest mit bis zu 4 C-Atomen bedeutet.

3. Verfahren nach den Ansprüchen 1 und 2, dadurch gekennzeichnet, daß man mit 3(2H)-Benzoxazolnonansulfonsäure-2-[4-(1,3-dimethyltetrahydro-2,4,6-trioxo-5(2H)-pyrimidinyliden-2-butenyliden]-Natriumsalz inkubiert.

4. Verfahren nach den Ansprüchen 1—3, dadurch gekennzeichnet, daß man die Leukozyten in isotonischer Salzlösung mit 1,5—30% autologem Plasma suspendiert, mit 10—30 μg/ml des Farbstoffs inkubiert, anschließend mit Trypanblau versetzt, zentrifugiert und fluoreszenzmikroskopisch auswertet.

## Claims

1. A method for determining the presence of leukemic cells characterised in that leukocytes are incubated in the presence of a dye of the general formula:

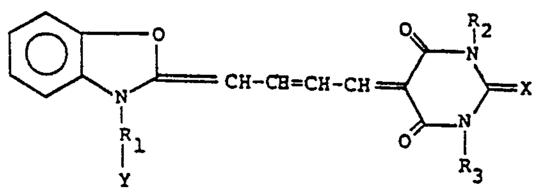

wherein
X is O or S;
Y is sulfo, sulfoxy, sulfino, sulfinoxy, phosphonoxy, carboxy, or the corresponding salts thereof;
$R_1$ is alkylene of 1—15 carbon atoms;
$R_2$ and $R_3$ are alkyl of 1—10 carbon atoms which can be branched; and the detection is carried out using a fluorescence microscope.

2. The method of Claim 1, characterised in that the incubation is carried out with a dye of the general formula, wherein X is O, Y is a sulfonic acid residue, $R_1$ is alkylene of 1—9 carbon atoms and $R_2$ and $R_3$ are linear or branched alkyl of up to 4 carbon atoms.

3. The method of Claims 1 and 2, characterised in that the incubation is carried out with 3(2H)-benzoxazole nonanesulfonic acid 2-[4-(1,3-dimethyl-tetrahydro-2,4,6-trioxo-5(2H)-pyrimidinylidene-2-butenylidene] sodium salt.

4. The method of Claims 1 to 3, characterised in that the leucocytes are suspended in an isotonic salt solution with 1,5—30% autologous plasma, incubated with 10—30 μg/ml of the dye, treated with trypan blue, and evaluated by a fluorescence microscope after centrifugation.

## Revendications

1. Procédé pour la détermination de cellules leucémiques, caractérisé en ce que l'on soumet les leucocytes à incubation avec un colorant de formule générale

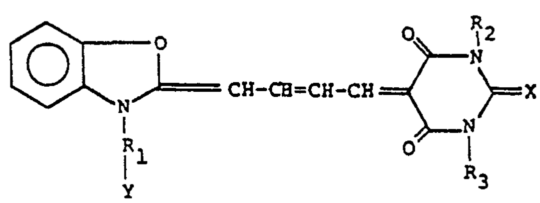

dans laquelle
X = O ou S,
Y = sulfo, sulfoxy, sulfino, sulfinoxy, phosphonoxy, carboxy ou les sels correspondants,
$R_1$ = alkylène en C 1—C 15
$R_2$ et $R_3$ = groupes alkyles en C 1—C 10 éventuellement ramifiés,
et on examine au microscope à fluorescence.

2. Procédé selon la revendication 1, caractérisé en ce que l'on soumet à incubation avec un colorant répondant à la formule générale dans laquelle X = O, Y = reste d'acide sulfonique, $R_1$ =

alkylène en C 1—C 9 et $R_2$ et $R_3$ = groupes alkyles linéaires ou ramifiés contenant jusqu'à 4 atomes de carbone.

3. Procédé selon les revendications 1 et 2, caractérisé en ce que l'on soumet à incubation avec le sel de sodium de l'acide 2-[4-(1,3-diméthyltétrahydro-2,4,6-trioxo-5(2H)-pyrimidinylidène-2-buténylidène]-3(2H)-benzoxazole-nonane-sulfonique.

4. Procédé selon l'une des revendications 1 à 3, caractérisé en ce que l'on met les leucocytes en suspension dans une solution saline isotonique avec 1,5 à 30% de plasma autologue, on soumet à incubation avec 10 à 30 microgrammes/ml du colorant, on ajoute ensuite du Bleu Trypan, on centrifuge et on examine au microscope à fluorescence.